# EUROPÄISCHE PATENTSCHRIFT

(11) **EP 0 796 834 B1**
(45) Veröffentlichungstag und Bekanntmachung des Hinweises auf die Patenterteilung: **30.08.2000**
(21) Anmeldenummer: 97103940.9
(22) Anmeldetag: 10.03.1997
(51) Int. Cl.: C07C 37/72, C07C 39/235

(54) **Reinigung von p.p-Bisphenolat-Lösungen**
Purification of p,p-bisphenolate solutions
Purification des solutions de p,p-bisphénolate

(30) Priorität: 21.03.1996 DE 19611141
(43) Veröffentlichungstag der Anmeldung: 24.09.1997
(73) Patentinhaber: BAYER AG, 51368 Leverkusen (DE)
(72) Erfinder: Wulff, Claus, Dr., 47800 Krefeld (DE); Meurer, Kurt-Peter, Dr., 51375 Leverkusen (DE); van Osselaer, Tony, Dr., 9100 Belsele (BE); Boyens, Roelof, 2100 Deurne (BE); Hinz, Jürgen, Dr., 47800 Krefeld (DE)

(56) Entgegenhaltungen:
- EP-A- 0 758 636
- WO-A-92/00943
- US-A- 4 400 553
- CHEMICAL ABSTRACTS, vol. 119, no. 26, 27.Dezember 1993 Columbus, Ohio, US; abstract no. 271942g, Seite 16; XP002052172 & CN 1 066 648 A (SHI MINGSHUI)

## Beschreibung

Die Erfindung betrifft ein Verfahren zur Reinigung von p,p-Bisphenolat-Lösungen, wie sie bei der Herstellung von Bisphenolaten erhalten werden aus Natronlauge und Bisphenolen durch Extraktion.

Die Herstellung hochreiner p,p-Natriumbisphenolat-Lösungen (NaBPA) ist bekannt. Durch mehrfache Umkristallisation des Bisphenol-A oder durch Destillation der Bisphenol-A-Schmelze gelingt es, hochreines Bisphenol-A herzustellen und anschließend kontinuierlich durch Umsetzung äquivalenter Mengen Bisphenol-A mit Natronlauge hochreine Natriumbisphenolat-Lösungen zu erzeugen (z.B. DE-A 4 413 396).

Es wurde nun gefunden, daß man p,p-Bisphenolat-Lösungen mit halogenfreien organischen Lösungsmitteln in der Weise extrahieren kann, daß die extrahierte p,p-Bisphenolat-Lösung weniger als 0,1 Gew.-% Isomere des korrespondierenden Bisphenols enthält.

Gegenstand der Erfindung ist ein Verfahren zur Reinigung von 14 bis 18 gew.-%igen phenolischen p,p-Bisphenolat-Lösungen, die bei der Herstellung von p,p-Bisphenolen erhalten werden, dadurch gekennzeichnet, daß in der p,p-Bisphenolat-Lösung mit einem Gehalt von mindestens 99,5 Gew.-% p,p-Bisphenolat durch extraktive Entfernung mit einem halogenfreien organischen Lösungsmittel die Isomeren des Bisphenols extrahiert und der Gehalt an p,p-Bisphenolat auf > 99,95 Gew.-% gebracht wird, bezogen auf gelösten Feststoff.

Die erfindungsgemäß zu reinigenden phenolischen p,p-Bisphenolat-Lösungen enthalten die bekannten bei der Herstellung von Bisphenol-A aus Phenol und Aceton ebenfalls entstehenden Nebenprodukte wie Isomere des Bisphenols-A, Indane und Alkyl-Phenolate. Diese p,p-Bisphenolat-Lösung wird erhalten durch Zusatz von Alkali (z.B. NaOH) zu der bei der Bisphenol-A-Herstellung erhaltenen umgesetzten Reaktionslösung.

Die erfindungsgemäß durch Extraktion zu reinigende p,p-Bisphenolat-Lösung hat eine Konzentration von 14 bis 18 Gew.-% p,p-Bisphenolat, bevorzugt 14,5 Gew.-%. Vorzugsweise liegt das p,p-Bisphenolat als Na-Salz (NaBPA) vor.

Die Extraktion kann kontinuierlich oder diskontinuierlich, vorzugsweise kontinuierlich, z.B. in Mixer-Settler-Apparaturen durchgeführt werden.

Als Extraktionsmittel werden halogenfreie, organische Lösungsmittel eingesetzt wie Ketone (z.B. Methylisobutylketon) und Ester wie Essigsäureethylester, vorzugsweise Essigsäureethylester.

Das Extraktionsmittel kann vom Extrakt z.B. durch Destillation abgetrennt und wiederverwendet werden. Ein so erhaltener Extrakt-Rückstand enthält die bei der Umsetzung von Phenol mit Aceton zu Bisphenol-A angefallenen hochsiedenden Isomeren. Diese Isomeren (Isomerenrückstand) können z.B. an Ionenaustauschern isomerisiert werden oder zusammen mit Restharzen der Bisphenol-A-Herstellung aus dem Prozeß ausgeschleust werden.

### Beispiele

### Beispiel 1

Es wurde eine 14,5 %ige NaBPA-Lösung mit einem Gehalt an p,p-BPA von 99,51 Gew.-% hergestellt. In der alkalischen Lösung lagen weiterhin 66 ppm Phenol, 89 ppm 4-tert.-Butylphenyl, 2160 ppm o,p-BPA, 99 ppm p,p-MethylBPA, 39 ppm Indane, 223 ppm Trisphenol und 1790 ppm Mol 402 vor.

Eine Volumeneinheit einer so hergestellten 14,5 %igen Natriumbisphenolatlösung mit einem OH^{―}-Überschuß von 0,20 % wurde 1 Minute lang mit der äquivalenten Volumeneinheit Essigsäureethylester viermal extrahiert. Die Phasen wurden abgetrennt, der BPA-Gehalt in der wäßrigen und in der organischen Phase GC-chromatographisch bestimmt.

In der wäßrigen Phase wurde eine BPA-Reinheit von 99,96 Gew.-% p,p-BPA GC-chromatographisch ermittelt. Der Anteil an Mol 402 betrug 20 ppm, an Trisphenol 108 ppm, an Indanen < 20 ppm, an p,p-MethylBPA < 30 ppm, an o,p-BPA 11 ppm, an 4-tert.-Butylphenol < 10 ppm und an Phenol 54 ppm.

### Beispiel 2

Wie in Beispiel 1 mit dem Unterschied, daß anstelle von Essigsäureethylester Methylisobutylketon eingesetzt wurde.

In der wäßrigen Phase wurde eine BPA-Reinheit von 99,89 Gew.-% p,p-BPA, 54 ppm Phenol, < 10 ppm 4-tert.-Butylphenol, 508 ppm o,p-BPA, 80 ppm p,p-MethylBPA, 27 ppm Indane, 180 ppm Trisphenol und 108 ppm Mol 402 gefunden.

### Vergleichsbeispiel A

Wie in Beispiel 1 mit dem Unterschied, daß als Lösungsmittel Chlorbenzol eingesetzt wurde.

Es konnten keine Isomeren (Nachweisgrenze) extrahiert werden.

### Vergleichsbeispiel B

Wie in Beispiel 1 mit dem Unterschied, daß als Lösungsmittel Chlorbenzol/Dichlormethan (50/50) eingesetzt wurde.

Es konnten keine Isomeren (Nachweisgrenze) extrahiert werden.

## Patentansprüche

1. Verfahren zur Reinigung von 14 bis 18 gew.-%igen phenolischen p,p-Bisphenolat-Lösungen, die bei der Herstellung von p,p-Bisphenolen erhalten werden, dadurch gekennzeichnet, daß in der p,p-Bisphenolat-Lösung mit einem Gehalt von mindestens 99,5 Gew.-% p,p-Bisphenolat durch extraktive Entfernung mit einem halogenfreien organischen Lösungsmittel die Isomeren der Bisphenole extrahiert und der Gehalt an p,p-Bisphenolat auf > 99,95 Gew.-% gebracht wird, bezogen auf gelösten Feststoff.

## Claims

1. Process for the purification of 14 to 18 wt.% phenolic p,p-bisphenolate solutions obtained during the preparation of p,p-bisphenols, characterised in that, in the p,p-bisphenolate solution containing at least 99.5 wt.% p,p-bisphenolate, the isomers of the bisphenols are extracted by extractive removal using a halogen-free organic solvent and the content of p,p-bisphenolate is brought to > 99.95 wt.%, based on dissolved solid.

## Revendications

1. Procédé pour la purification de solutions phénoliques de p,p-bisphénolate à de 14 à 18 % en poids, lesquelles sont obtenues lors de la préparation de p,p-bisphénols, caractérisé en ce que l'on extrait dans la solution de p,p-bisphénolate avec une teneur d'au moins 99,5% en poids de p,p-bisphénolate les isomères des bisphénols par élimination extractive avec un solvant organique exempt d'halogène et on amène la teneur en p,p-bisphénolate à >99,95% en poids, rapportés à la matière solide dissoute.
